Europäisches Patentamt

European Patent Office

Office européen des brevets

(11) Numéro de publication: **0 048 011**
**B1**

(12) **FASCICULE DE BREVET EUROPEEN**

(45) Date de publication du fascicule du brevet: **26.02.86**

(51) Int. Cl.⁴: **A 41 B 13/02**

(21) Numéro de dépôt: **81107224.8**

(22) Date de dépôt: **14.09.81**

(54) Couche-culotte et procédé de fabrication de telles couches-culottes.

(30) Priorité: **15.09.80 FR 8019861**

(43) Date de publication de la demande:
**24.03.82 Bulletin 82/12**

(45) Mention de la délivrance du brevet:
**26.02.86 Bulletin 86/09**

(84) Etats contractants désignés:
**AT BE CH DE GB IT LI**

(56) Documents cités:
**EP-A-0 023 804**
**DE-A-2 649 948**
**FR-A-1 600 732**
**FR-A-2 082 803**
**FR-A-2 177 425**
**FR-A-2 421 571**
**GB-A-2 041 224**
**US-A-2 834 347**
**US-A-3 644 157**
**US-A-4 205 679**

(73) Titulaire: **BOUSSAC SAINT FRERES B.S.F.**
**Société anonyme dite:**
**12, rue du Vieux Faubourg**
**F-59800 Lille (FR)**

(72) Inventeur: **de Jackheere, Raphael**
**797, Domaine de la Vigne**
**F-59910 Bondues (FR)**
Inventeur: **Dussaud, Jacques**
**92, rue Jacquemars Gielée**
**F-59800 Lille (FR)**

(74) Mandataire: **Casalonga, Alain et al**
**BUREAU D.A. CASALONGA OFFICE JOSSE &**
**PETIT Baaderstrasse 12-14**
**D-8000 München 5 (DE)**

Courier Press, Leamington Spa, England.

## Description

La présente invention se rapporte à une couche-culotte comprenant une enveloppe extérieure constituée par un matériau souple en feuille, imperméable à l'humidité, cette enveloppe étant garnie intérieurement d'un coussin absorbant de forme allongée. Le coussin absorbant fait partie intégrante de l'ensemble qui peut être jeté après usage et que l'on appelle également "changes complet". De tels produits d'hygiène peuvent être utilisés pour des enfants en bas âge ou pour des personnes adultes incontinentes.

L'invention a également trait à un procédé de fabrication de telles couches-culottes.

Les couches-culottes de ce type se présentent à l'heure actuelle généralement sous la forme de culottes ouvertes, c'est-à-dire divisées sur les deux côtés, entre la ceinture et chaque ouverture de passage pour les jambes. La fermeture de la culotte s'effectue par des systèmes de ruban adhésif permettant de relier la partie avant de la culotte à la partie arrière, sur les deux côtés de la culotte. A titre d'exemple de telles couches-culottes, on peut citer le brevet U.S. 3.180.335.

Pour permettre un maintien correct de la culotte sur le bébé ou l'utilisateur, il est nécessaire de serrer la ceinture de la culotte à la taille. Or, il est difficile de régler correctement ce serrage de la ceinture lors de la pose de la culotte puisque ce serrage s'effectue en deux temps, sur les deux côtés opposés de la culotte. C'est pourquoi, il est souvent nécessaire d'ouvrir de nouveau la culotte sur un côté au moins, pour réajuster ce serrage. Or, il s'avère que les systèmes de fermeture par adhésif se déchirent ou s'arrachent souvent de la culotte lors d'une telle ouverture, ce qui rend la couche-culotte inutilisable.

On a déjà prévu dans le brevet français N°. 2082803, d'introduire dans un ourlet, à la taille d'une culotte à jeter en matière plastique pouvant recevoir ultérieurement une garniture absorbante qui ne fait pas partie intégrante de la culotte, un lien qui peut ensuite être serré à partir d'ouvertures pratiquées dans l'ourlet. Malgré la suppression des rubans adhésifs, l'existence de ce lien à la taille ne permet pas dans la pratique une utilisation aisée.

Le brevet français FR—A—1.600.732 décrit une couche-culotte dont les pourtours de ceinture et de passage des jambes sont 'travaillés" pour les rendre élastiques. Des soudures latérales sont prévues pour fermer la culotte en recouvrant deux épaisseurs de l'enveloppe imperméable. Aucune indication n'est fournie dans ce document sur une fabrication industrielle d'une telle couche-culotte.

Le brevet US—A—3.644.157 décrit un procédé de fabrication d'une culotte dénuée de matelas absorbant et d'élastique de ceinture continu. La soudure latérale fermant la culotte se fait sur tois épaisseurs en recouvrement de l'enveloppe.

Jusqu'à présent, comme décrit par exemple dans le brevet français FR—A—2.086.605, la fabrication de couches-culottes en continu était généralement réalisée par une succession d'opérations sur un matériau souple en feuille se déroulant à grande vitesse et sur lequel des coussins absorbants de forme allongée étaient posés longitudinalement, les découpes pour les passages des jambes étant faites sur les portions marginales de la feuille.

La demande de brevet DE—A—2.649.948 montre un procédé de ce type, complété par la pose d'élastiques rectilignes collés selon l'axe de déroulement de la feuille composite qui est ensuite découpée transversalement pour former les couches-culottes.

Dans certains cas, comme décrit dans le brevet français FR—A—2.082.803, on a pu prévoir au contraire une disposition inverse, le pliage final des produits découpés se faisant selon une ligne longitudinale. Toutefois, dans ce cas, il n'était pas possible d'envisager la pose, à l'état tendu, d'éléments élastiques destinés à améliorer l'étanchéité au passage des jambes. Dans ces conditions, les couches-culottes munies d'éléments élastiques à l'entre jambe, telles que décrites par exemple dans les brevets US—A— 3.860.003 et US—A—4.050.462, dans lesquelles ces éléments élastiques étaient essentiellement rectilignes, ne pouvaient être fabriquées en continu que selon le premier procédé précité.

La réalisation de passages de jambes curvilignes suivant le bord de ces passages, tel que décrit par exemple dans le brevet français FR—A—2.063.794 pour une couche-culotte pouvant recevoir un coussin absorbant, ne pouvait donc être réalisée de manière industrielle en continu et à grande cadence. Malgré l'avantage que présentent de tels élastiques curvilignes, on se trouvait donc réduit jusqu'à présent, à l'utilisation d'élastiques d'entre jambes rectilignes.

L'invention a pour objet un procédé de fabrication permettant de réaliser de façon simple et à grande cadence de telles couches-culottes. Le procédé de l'invention permet en outre d'utiliser des éléments élastiques curvilignes afin d'améliorer l'étanchéité au passage des jambes.

Tel qu'il est revendiqué, le procédé de fabrication de couches-culottes suivant l'invention, comprend les étapes suivantes: on déroule une feuille souple continue d'un matériau thermosoudable imperméable à l'humidité; on colle à l'état étiré, sur au moins la majeure partie de la longueur de ladite feuille, un élastique continu; on pose sur la feuille imperméable, à intervalles réguliers, des coussins absorbants de forme allongée, de manière que l'axe longitudinal des coussins soit perpendiculaire à l'axe longitudinal de ladite feuille; on déroule sur la feuille imperméable ainsi garnie de coussins, un voile continu d'un matériau thermo-soudable mince, perméable à l'humidité de façon à obtenir une bande composite; on découpe dans ladite bande composite, entre chaque fois deux coussins successifs, entre les deux bords longitudinaux, une pluralité d'ouvertures symétriques par rapport à une ligne transversale passant perpendiculairement auxdits bords, à mi-distance entre les coussins successifs; on plie la bande composite précitée suivant une ligne médiane; et on sectionne, par une opération combinée de soudage et de découpage à chaud, la bande composite pliée, suivant la ligne transversale précitée.

Les deux extrémités des élastiques de taille peuvent être soudées les unes aux autres lors de l'opération de soudure précitée. Dans une variante, la portion de chaque élastique coupant la ligne transversale précitée n'est pas collée sur la feuille imperméable. Lors de la découpe selon la ligne transversale précitée, les élastiques de taille sont sectionnés et leurs extrémités non collées se rétractant quelque peu jusqu'à la portion collee de l'élastique.

La feuille imperméable est avantageusement encollée avant la pose des des coussins absorbants. Cet encollage peut se faire, par exemple, par des lignes de colles continues ou discontinues, espacées transversalement sur la feuille.

Afin d'éviter le contact de la peau du bébé ou de l'utilisateur avec la feuille imperméable, il est avantageux de dérouler sur la feuille déjà garnie de coussins absorbants, avant que ne soient pratiquées lesdites ouvertures, un voile continu d'un matériau thermo-soudable mince, perméable à l'humidité, par exemple un voile de non-tissé thermofusible. Ce voile peut être préencollé, de manière à adhérer à la fois au coussin absorbant et à la feuille imperméable aux endroits où cette dernière n'est pas garnie de coussins absorbants.

Lorsqu'on pratique ensuite lesdites ouvertures entre les coussins absorbants, ces ouvertures sont ménagées non seulement dans le feuille imperméable, mais également dans le voile perméable. Le voile perméable et la feuille imperméable sont maintenus collés sur les bords des ouvertures en raison des lignes de colle assurant la fixation respective de ces deux feuilles.

Dans un mode de réalisation avantageux, on peut procéder à un découpage à chaud pour pratiquer ces ouvertures, ce qui entraîne un soudage simultané du voile à la feuille sur le bord des ouvertures.

Le sectionnement transversal à chaud de la feuille pliée, entre les coussins absorbants, entraîne simultanément un soudage des deux plis du voile et des deux plis de la feuille de part et d'autre de chaque ligne transversale de sectionnement.

Lorsque la couche-culotte doit être munie d'élastiques d'entre jambe à la hauteur des ouvertures de passage pour les jambes, afin d'assurer une meilleure étanchéité à cet endroit, ces élastiques sont avantageusement appliqués sur la feuille avant la pose des coussins absorbants sur cette dernière.

Suivant un mode de réalisation particulièrement simple, on déroule deux élastiques continus sous tension par exemple sous forme de bandelettes minces au-dessus de la feuille de manière qu'ils passent de part et d'autre des ouvertures pratiquées ultérieurement dans la feuille. On encolle préalablement les deux élastiques au moins sur une partie de leur surface de contact de manière que lors de leur application sur la feuille, ils viennent se coller sur cette dernière au voisinage du bord des dites ouvertures. On sectionne ensuite les deux élastiques, après leur collage sur la feuille, entre deux ouverture successives, de manière à supprimer ou simplement détendre les portions non encollées des élastiques sur les zones de la feuille destinées à recevoir les coussins absorbants.

Dans un mode de réalisation préféré, on déplace périodiquement dans une direction transversale, le point de contact initial entre la surface encollée de chaque élastique et la feuille imperméable en mouvement de façon à obtenir un élastique d'entre jambe de forme incurvée, collé sur la feuille imperméable le long d'au moins une porition courbe de chaque ouverture de passage de jambe.

Le point de contact initial est avantageusement maintenu fixe par intermittance de façon à définir des portions rectilignes, de préférence correspondant aux portions non encollées, destinées à être ultérieurement découpées et détendues ou supprimées.

Dans un autre mode de réalisation, on dépose sur la feuille imperméable, autour du bord de chaque ouverture de passage de jambe, une bande d'une matière thermoplastique et adhésive en fusion, capable de se rétracter lors de son refroidissement et possèdant une bonne élasticité à l'état froid. Il est ainsi possible d'obtenir également la formation d'éléments élastiques curvilignes autour des ouvertures de passage de jambe.

Pour la fabrication de couches-culottes comportant des fils de déchirure pour faciliter l'enlèvement de la couche culotte après usage, on applique sur la feuille ou sur le voile, avant le pliage longitudinal, de part et d'autre de la ligne transversale de sectionnement ultérieur, un fil de déchirure en matière thermo-fusible, entre un bord longitudinal de la feuille et chaque ouverture de passage de jambe.

Il est possible d'utiliser à cet effet, un fil de déchirure encollé ou de former le dit fil par extrusion d'un fil de matière plastique en fusion sur la feuille ou sur le voile.

La couche-culotte à jeter conforme à l'invention, comprend une enveloppe extérieure constituée par un matériau souple en feuille, imperméable à l'humidité, garni intérieurement d'un coussin absorbant de forme allongée faisant partie intégrante de la couche-culotte et recouvert de préférence d'un voile intérieur perméable à l'humidité, l'ensemble étant assemblé de façon que la couche-culotte se présente sous la forme d'un slip fermé comportant un élastique de serrage entourant la taille sur au moins une majeure partie de sont pourtour. Les côtés latéraux sont fermés par au moins une soudure s'etendant depuis la ceinture jusqu'à l'ouverture de passage des jambes, en disposant les deux côtés sensiblement bord à bord.

Du fait de la présence de l'élastique sans fin à la taille du slip complètement fermé, la couche-culotte peut être enfilée sans problème et procure un serrage à la taille assurant un maintien correct, même sur des utilisateurs ayant des tours de taille différents.

Du préférence, la couche-culotte conforme à l'invention comprend également des éléments élastiques disposés autour d'au moins une partie des ouvertures de passage pour les jambes, afin d'assurer une bonne étanchéité à cet endroit.

Pour enlever la couche-culotte après usage, il est possible de la déchirer d'un côté ou sur les deux côtés, ce qui ne présente pas de difficulté compte tenu de la faible épaisseur de l'enveloppe extérieure de la couche-culotte. Cependant, pour simplifier ce déchirement, la couche-culotte comporte de préférence au moins un fil de déchirure intérieur solidaire de la culotte et s'étendant entre la ceinture et les ouvertures de passage pour les jambes.

Le profil de chaque ouverture de passage de jambe est avantageusement convexe sur sa portion arrière et concave sur sa portion avant. L'avant et l'arrière doivent être compris comme s'entendant des faces avant et arrière de la couche-culotte portée par le bébé ou l'utilisateur.

Selon un premier mode de réalisation préféré, ces profils sont courbes et un élastique d'entre jambe entoure chaque profil sur au moins une majeure partie de sa périphérie.

Selon un autre mode de réalisation, ces profils sont délimités par une ligne brisée comportant au moins une section parallèle à l'élastique de taille. Des portions d'élastiques, par exemple en forme de bandelette, sont alors collées le long de ces sections rectilignes.

Dans tous les case, on obtient ainsi un profil du passage des jambes, se rapprochant de l'anatomie du bébé ou de l'utilisateur et assurant, grâce aux éléments élastiques, une excellente étanchéité à cet endroit.

Le coussin absorbant peut être de forme sensiblement rectangulaire ou, selon une variante, affecter la forme d'un sablier.

En se référant aux dessins annexes, on va décrire ci-après plus en détail à titre d'exemples nullement limitatifs, plusieurs modes de réalisation d'une couche-culotte conforme à l'invention et plusieurs modes de réalisation d'un procédé de fabrication de telles couches-culottes. Sur les dessins:

— la figure 1 représente une couche-culotte conforme à l'invention sur un bébé;
— la figure 2 représente certaines phases de fabrication d'une couche-culotte suivant la figure 1;
— la figure 3 représente une phase de fabrication ultérieure de la couche-culotte selon la figure 1;
— la figure 4 représente certaines phases d'une variante du procédé de fabrication selon l'invention;
— la figure 5 représente une autre variante du procédé de fabrication de l'invention;
— la figure 6 représente encore une autre variante de procédé de fabrication de l'invention;
— la figure 7 représente en élévation une couche-culotte obtenue par la mise en oeuvre du procédé illustré sur la figure 6;
— la figure 8 représente une variante supplémentaire du procédé de fabrication de l'invention;
— et la figure 9 représente en élévation une couche-culotte obtenue par la mise en oeuvre du procédé illustré sur la figure 8.

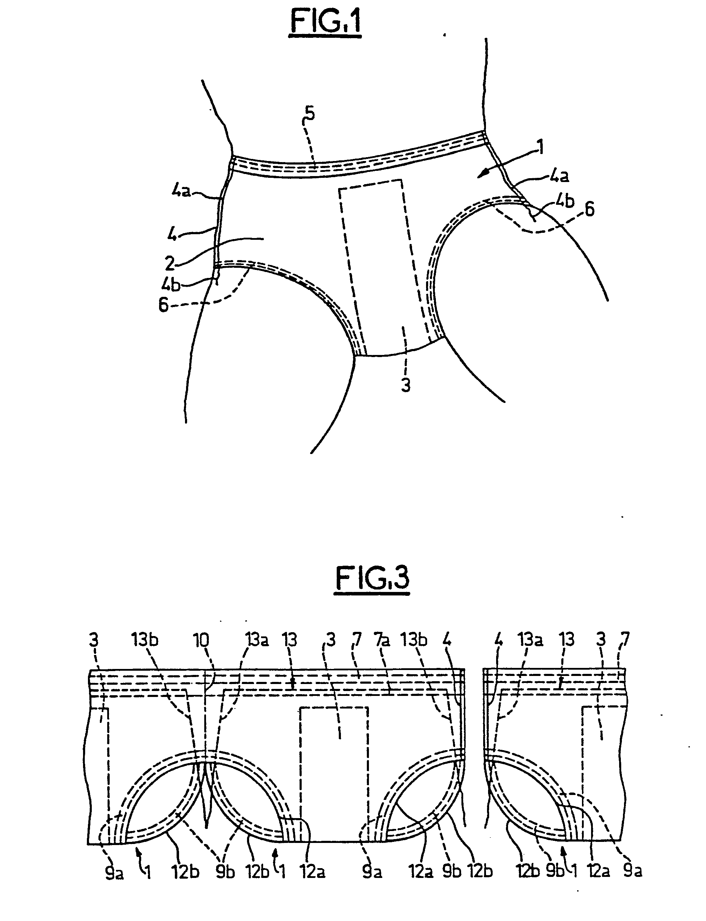

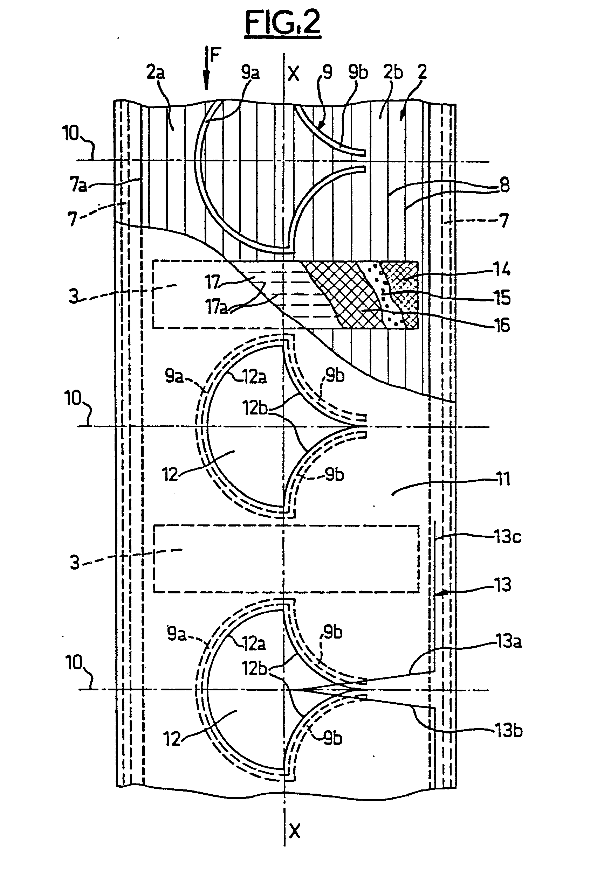

Sur la figure 1, on reconnait une couche-culotte 1, avec une enveloppe extérieure 2, formée d'un matériau souple en feuille, imperméable à l'humidité, par exemple une feuille de polyéthylène. La feuille 2, est garnie intérieurement d'un coussin absorbant 3 de forme rectangulaire allongée. La feuille 2 se présente sous la forme d'un slip fermé sur les deux côtés latéraux par une soudure 4 s'étendant de la ceinture jusqu'à l'ouverture de passage pour la jambe. La ceinture du slip est garnie d'un élastique 5 monté dans un ourlet.

Le slip peut comporter intérieurement, à la hauteur des ouvertures de passage pour les jambes, des éléments élastiques 6 assurant une meilleure étanchéité à cet endroit et collés le long de la majeure partie de la périphérie curviligne de chaque ouverture de passage de jambe.

Pour utiliser une teile couche-culotte, on l'enfile à la manière d'un slip, en passant les jambes dans les ouvertures de passage précitées. Pour retirer la couche-culotte après usage, il est facile de déchirer l'enveloppe extérieure au droit des côtés latéraux de la couche-culotte. Pour faciliter cette opération, la couche-culotte 1, présente cependant deux fils de déchirure 4a dont les extrémités 4b peuvent être saisies afin de déchirer les côtés latéraux par une simple traction sur les fils 4a.

La figure 2, représente certaines phases de la fabrication d'une couche-culotte suivant la figure 1. On reconnait la feuille 2 destinée à former l'enveloppe extérieure des couches-culottes en constituée par une bande continue de polyéthylène se déplaçant dans le sens de la flèche F selon son axe longitudinal X—X.

Une bandelette élastique 7, continue, est collée sur toute la longueur de chaque bord longitudinal de la feuille 2, à l'état étiré. Le collage peut se faire soit de manière continue soit sur la majeure partie de la bandelette élastique 7. Dans ce dernier cas, on évite notamment le collage des portions des bandelettes 7 coupant la ligne transversale de symétrie 10.

Chaque bord longitudinal de la feuille 2, est rabattu autour de l'élastique 7 et fixé à la feuille 2 par une ligne de soudure longitudinale 7a, pour constituer ainsi un ourlet entourant l'élastique 7. La feuille 2, est munie de plusieurs lignes d'encollage longitudinales 8 parallèles et espacées. A ce stade de la fabrication, représenté par la partie haute de la figure 2, des coussins absorbants 3, sont posés à intervalles réguliers sur la feuille 2 et se trouvent collés par les lignes d'encollage 8. Les coussins 3 présentent une forme allongée, leur axe longitudinal étant perpendiculaire à l'axe longitudinal X—X de la feuille 2.

Une bande d'un matériau élastique 9 est posée entre les coussins 3 et selon un profil curviligne autour d'une zone destinée à être ultérieurement découpée pour former des passages pour les jambes. Du côté 2a de l'axe longitudinal X—X, qui correspondra à la partie avant des couches-culottes, le profil 9a de la bande 9 est concave et circulaire. L'arc de cercle couple à angle droit la ligne transversale de symétrie 10. Du côté 2b opposé, correspondant à la partie arrière de la couche-culotte terminée, le profil 9b est, convexe et comprend deux arcs de cercle venant tangenter l'axe X—X et la ligne transversale de symétrie 10. L'ensemble du profil 9 est symétrique par rapport à la ligne transversale 10. Le caractère concave ou convexe des profils est apprécié par rapport à l'ouverture ultérieure.

La bande de matériau élastique 9 est obtenue par exemple par dépose d'une pâte élastique visqueuse à chaud et présentant à froid des caractéristiques d'élasticité convenables. Des matériaux à base de latex peuvent notamment convenir pour une telle utilisation. Un voile mince 11 perméable à l'humidité, par exemple en non tissé de polypropylène est ensuite déroulé sur la feuille 2 garnie des coussins absorbants 3, le voile 11 étant encollé préalablement à son application sur sa face interne de manière à adhérer à la feuille 2, et aux coussins 3.

On réalise ensuite, comme représenté dans la partie médiane de la fig 2, entre chaque fois deux coussins successifs 3, des ouvertures 12 par une opération simultanée ou des opérations successives de soudage à chaud et de découpe au cours desquelles le voile 11 est soudé à la feuille 2 sur le contour curviligne des ouvertures 12 puis découpé suivant le même contour. Il est également possible, en variante, de se contenter d'une opération unique de découpe. Le voile 11 reste alors collé sur la feuille 2 même sur les bords des ouvertures 12, en particulier par les lignes d'encollage 8.

On notera que chaque ouverture 12 est symétrique par rapport à la ligne transversale 10 passant à mi-distance entre deux coussins 3 respectifs. Chaque ouverture 12 comprend une portion concave circulaire 12a et deux portions convexes 12b qui sont respectivement bordées par les portions curvilignes 9a, 9b de la bande élastique 9.

Un fil de déchirure 13 est ensuite appliqué sur le voile 11 du côté arrière de la couche-culotte terminée. Le fil 13 suit, à l'endroit de chaque ouverture 12, un tracé en V 13a, 13b de part et d'autre de la ligne 10, la pointe du V pénétrant dans l'ouverture 12. Entre deux tracés successifs en V, le fil 13 suit un trajet rectiligne 13c dans le sens longitudinal de la bande 2, à proximité d'un bord longitudinal de cette dernière. Le fil 13 peut être simplement collé sur la bande composite continue ou, dans une variante, formé par extrusion d'un fil de matière thermoplastique en fusion directement sur ladite bande composite. La partie basse de la fig 2 représente l'état de la bande composite, à ce stade de la fabrication.

Sur la figure 2, on a également représenté, par des arrachement successifs, la constitution d'un coussin absorbant 3 à titre d'exemple. Dans le mode de réalisation illustré, chaque coussin absorbant 3 se compose de bas en haut, successivement, d'un ou de plusiuers plis 14 de ouate cellulose, d'une couche 15 d'un produit à grand pouvoir absorbant en poudre, en fibres ou en film, d'un ou de plusieurs plis 16 de ouate de cellulose et éventuellement d'une feuille 17 en un matériau imperméable à l'humidité présentant, au moins dans la zone de l'entrejambe, de fines fentes 17a de faible longueur, orientées dans le sens de la longueur du coussin absorbant 3. Les plis 14 et 16 de ouate de cellulose sont de préférence gauffrés en carré ou en losange.

Le coussin absorbant peut, dans un autre exemple, se composer depuis sa face inférieure jusqu'à sa face supérieure successivement, d'un ou du plusieurs plis de ouate de cellulose, d'une ou deux épaisseur de pulpe de cellulose broyée connue sous l'appellation de "fluff" de cellulose, d'un ou de plusieurs plis de ouate de cellulose et éventuellement d'une feuille imperméable munie de fentes, analogue à la feuille 17. Les coussins sont de préférence gauffrés en carré ou en losange.

La bande composite représentée dans le bas de la figure 2 est ensuite pliée suivant l'axe longitudinal médian X—X.

On obtient alors l'aspect représenté sur la gauche de la figure 3 où l'on remarque déjà la forme des différentes couches-culottes 1 qui sont reliées les unes aux autres par la ligne transversales 10. Les parties avant et arrière de chaque couche-culotte 1 comportant à la taille un élastique 7. Les passages de jambes présentent un profil curviligne suivant l'anatomie du bébé et définis à l'avant par une moitié des portions concaves circulaires 12a et sur la face arrière de la couche-culotte par une des portions convexes 12b.

Les bandes élastiques 9a et 9b sont disposées le long du profil curviligne des ouvertures 12 de façon à assurer la meilleure étanchéité possible à cet endroit.

Lors de l'étape finale du procédé de fabrication représentée sur la droite de la figure 3, on sectionne par une opération combinée de soudage et de découpage à chaud selon la ligne transversale de sectionnement 10 la bande composite repliée.

Dans cette opération, les différentes couches-culottes 1 se trouvent donc séparées les unes des autres. De plus, les côtés. Latéraux des couches-culottes 1 sont fermés par des soudures respectives 4 s'étendant depuis la ceinture jusqu'à l'ouverture 12 de passage pour les jambes. Dans le mode de réalisation représenté sur la figure 3, on a prévu deux lignes de soudure 4 disposées de part et d'autre de la ligne de sectionnement transversale 10. On pourrait bien entendu envisager dans une variante une soudure unique de plus grande largeur au milieu de laquelle on procèderait au sectionnement.

Lors de l'opération de découpage, on sectionne en outre la pointe du V du fil de déchirure 13 en laissant subsister les extrémités libres 13a et 13b qui constituent donc des bouts de préhension permettant de faciliter l'ouverture de la couche-culotte après son utilisation par déchirage des côtés latéraux.

Par ailleurs, lorsque les bandelettes 7 sont collées de manière continue, les soudures 4 solidarisent également les extrémités découpées des élastiques 7 disposés respectivement sur les côtés avant et arrière de la couche-culotte de sorte qu'après l'opération de découpage, la taille de la couche-culotte 1 se trouve munie d'un élastique continu. Dans la variante où les portions des bandelettes 7 coupant les lignes transversales 10 ne sont pas collées, l'opération finale de découpe provoque le sectionnement des élastiques 7 à cet endroit et leur rétraction sur une faible longueur jusqu'aux portions collées à l'intérieur de l'ourlet entourant l'élastique 7.

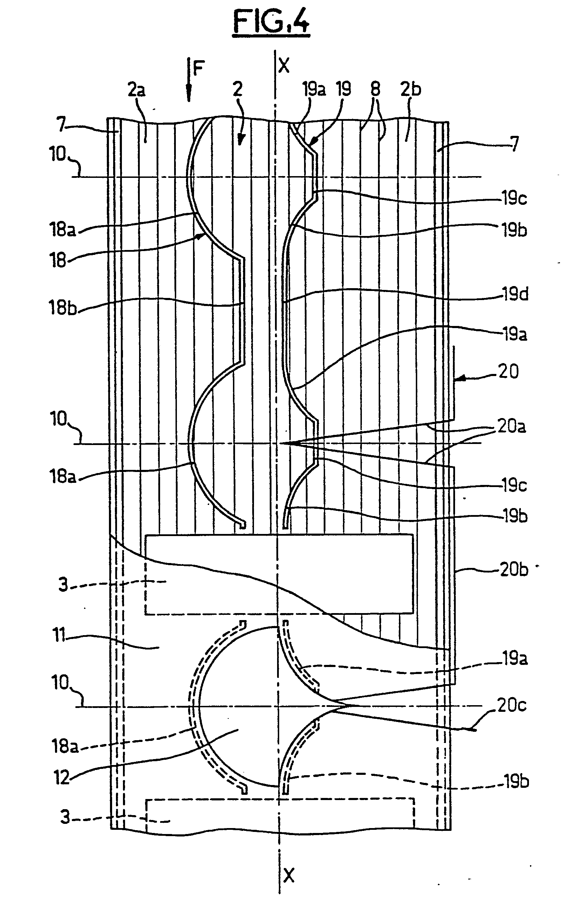

Le mode de réalisation illustré sur la figure 4 diffère uniquement de celui de la figure 2 par le mode de pose des éléments élastiques destinés à former les élastiques d'entrejambes des couches-culottes.

Dans ce mode de réalisation, où les éléments identiques portent les mêmes références, les bandelettes élastiques 7 continues sont également collées à l'état étiré sur toute la longueur de chaque bord longitudinal de la feuille 2 ou sur une majeure partie de cette longueur. Dans ce mode de réalisation, les élastiques 7 ne sont cependant pas logés dans des ourlets pratiqués ultérieurement par repli comme c'était le cas dans le mode de réalisation de la figure 2.

Les éléments élastiques d'entrejambes sont réalisés au moyen de deux bandelettes élastiques distinctes référencées respectivement 18 et 19 dans leur ensemble.

La bandelette élastique 18 est collée par intermittence sur la feuille imperméable 2 selon un trajet sinueux se trouvant par rapport à l'axe longitudinal X—X sur le côté correspondant à l'avant de la couche-culotte terminée. La bandelette élastique 19 est quant à elle collée par endroits selon un trajet sinueux sur la portion 2b de la feuille imperméable 2, du côté de l'axe longitudinal X—X qui correspond à l'arrière de la couche-culotte terminée.

Le trajet de la bandelette élastique 18 comporte des portions circulaires 18a concaves symétriques par rapport aux lignes transversales 10 et coupant ces dernières de manière perpendiculaire au sommet de l'arc de cercle. Les portions 18a sont collées sur toute la longueur de leur trajet curviligne sur la feuille imperméable 2.

Pour obtenir un tel collage, on déplace périodiquement dans une direction transversale, le point de contact initial de collage de la bandelette élastique 18 par rapport à la feuille imperméable 2. Le collage se fait de préférence au moment où la feuille imperméable 2 passe sur un cylindre métallique entraîné en rotation et avantageusement maintenu à une température relativement basse par exemple de l'ordre d'environ 20°C. Le choix de la température du cylindre dépend de paramètres tels que la température de l'adhésif liquide utilisé, la quantité d'adhésif déposé, la masse du cylindre lui-même. On fait en sorte que la différence de température entre l'adhésif liquide avant le contact avec la feuille 2 et le cylindre soit telle que l'adhésif liquide subisse un brusque refroidissement suffisant pour lui permettre de maintenir la bandelette élastique à l'encontre des efforts transversaux résultant de sa pose un trajet curviligne. De cette manière, la colle liquide qui a été déposée à haute température sur la surface de la bandelette élastique destinée à entrer en contact avec la feuille 2, réalise un collage immédiat dès le contact avec la feuille imperméable 2 en raison de la différence de température qui provoque la prise immédiate de la colle. Le déplacement périodique en direction transversale du point de contact initial permet donc l'obtention d'un élastique incurvé 18a collé à l'état tendu étiré, la courbure de cet élastique étant susceptible de suivre le profil de l'ouverture ultérieure de passage des jambes.

Les différentes portions curvilignes 10a collées sur la feuille imperméable 2 sont séparées par des portions rectilignes 18b non collées et disposées parallèlement à l'axe longitudinal X—X avec un certain écartement par rapport à ce dernier.

L'élastique 19 comporte des portions curvilignes 19a et 19b en forme d'arc de cercle disposées de manière convexe par rapport à la future ouverture du passage de jambes et collées sur la feuille imperméable 2 par le même procédé que les portions curvilignes 18a de la bandelette élastique 18. Les portions curvilignes 19a et 19b disposées de chaque côté de la ligne transversale 10 et symétriquement par rapport à cette dernière sont séparées par une portion rectiligne 19c non collée parallèle à l'axe longitudinal X—X à un certain écartement de ce dernier et coupant perpendiculairement la ligne transversale 10 dans une zone correspondant à la pointe de la future ouverture de passage d'entrejambes.

Le trajet de l'élastique 19 se complète par une portion rectiligne 19d qui n'est pas non plus collée sur la feuille 2 et disposée parallèlement à l'axe longitudinal X—X à proximité immédiat de ce dernier.

Avant la pose des coussins absorbants 3, on procède à la suppression par découpe des portions rectilignes non collées 18b et 19d des bandelettes élastiques 18 et 19. Pour ce faire, on peut par exemple par l'application d'un vide sur la surface inférieure de la feuille 2, réaliser un écartement momentané de cette dernière et des portions rectilignes 18b et 19d permettant d'effectuer deux découpes de l'élastique à l'aide d'un outil tranchant lors du déplacement en continu de l'ensemble selon l'axe X—X dans le sens de la flèche F.

Dans une variante on se contente d'effectuer une seule découpe sur les différentes portions non collées 18b et 19d ce qui provoque une rétraction de ces portions non collées et initialement étirées. On notera que les lignes d'encollage 8 constituées par un adhésif permanent ne sont pas suffisamment adhésives pour empêcher le relâchement de la partie non encollée d'une bandelette élastique. Il est cependant préférable, comme illustré sur la fig. 4, de ne pas superposer sur toute sa longueur une telle portion non encollée avec une des lignes d'encollage 8 afin de faciliter le relâchement après découpe.

Un fil de déchirure 20 est ensuite déposé sur la portion 2b de la feuille 2 en suivant un trajet comportant une série de V 20a symétriques par rapport aux lignes 10 et dont la pointe se poursuit dans la zone de l'ouverture future de passage de jambe. Les trajets rectilignes 20b qui relient entre eux les différents V 20a, se trouvent à l'extérieur du bord longitudinal de la feuille 2 du côté 2b correspondant à l'arrière de la couche-culotte.

On pose ensuite, comme représenté à la partie basse de la figure 4, les coussins absorbants 3 sur la feuille 2 entre les différentes zones correspondant aux futures ouvertures de passage des jambes c'est-à-dire aux endroits où l'on a préalablement supprimé ou détendu les portions rectilignes 18b et 19d des élastiques 18 et 19.

Le voile perméable 11 préalablement encollé partiellement, par exemple au moyen de lignes de collage, est ensuite déroulé par dessus l'ensemble et on procède au découpage des ouvertures 12 comme représenté dans la partie basse de la figure 4. On peut également souder comme précédemment la feuille 2 et le voile 11 sur les bords des ouvertures 12 simultanément ou au cours d'une opération distincte.

On notera que les ouvertures 12 présentent le même profil que celles de la figure 2 et que la découpe permet d'éliminer la portion rectiligne non collée 19c de l'élastique 19 ainsi que la pointe des V 20a des fils de déchirure 20. Ces derniers sont en outre sectionnés à l'extérieur de la feuille 2 de façon à ne laisser subsister que les extrémités libres 20c permettant de tirer sur le fil de déchirure et de procéder à l'ouverture de la couche-culotte. La fabrication se termine comme dans le mode de réalisation de la figure 2 par pliage autour de l'axe longitudinal X—X et découpe avec soudure simultanée au droit des lignes transversales 10.

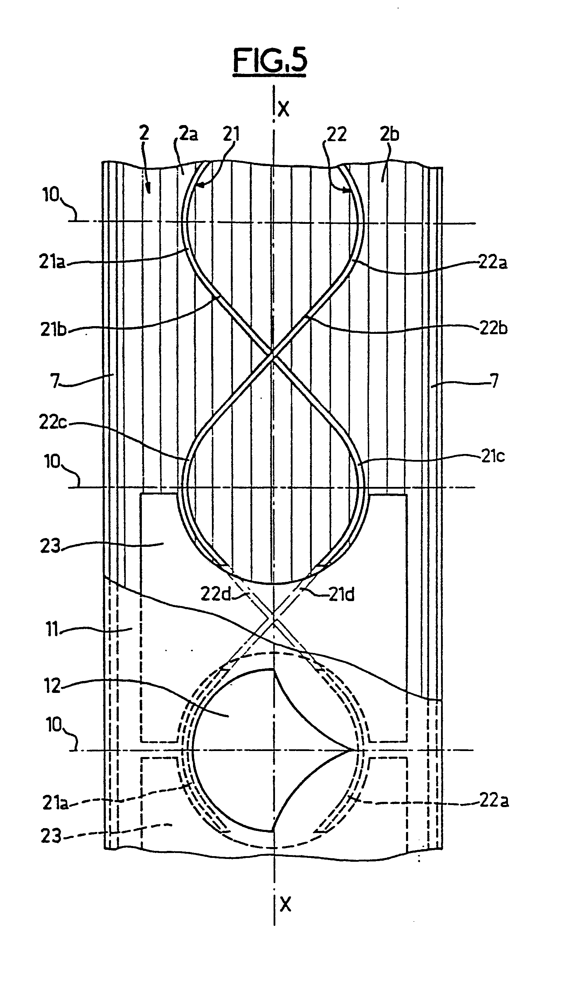

Dans le mode de réalisation illustré sur la figure 5 où les mêmes éléments portent les mêmes références, les bandelettes élastiques 21 et 22 destinés à réaliser les éléments élastiques d'entre-jambes sont collées alternativement d'un côté et de l'autre de l'axe longitudinal X—X en se croisant en un point voisin de cet axe.

Dans ces conditions, chaque bandelette élastique 21 comporte une portion de trajet incurvé en arc de cercle 21a se trouvant collé à l'état étiré sur la zone 2a correspondant à l'avant de la couche-culotte finale, puis une portion rectiligne 21b non collée coupant l'axe longitudinal X—X selon un angle d'environ 45° suivie d'une portion curviligne collée en arc de cercle 21c symétrique de la portion 2a et de même courbure mais se trouvant cette fois sur le côté 2b correspondant à l'arrière de la couche-culotte terminée. L'élastique 21 se poursuit par une portion rectiligne 21d coupant à nouveau l'axe longitudinal X—X pour revenir sur le côté 2a.

L'elastique 22 est symétrique de l'élastique 21 par rapport à l'axe longitudinal X—X et comporte donc successivement une portion curviligne circulaire 22a collée sur la portion 2b, suivie par une portion rectiligne 22b non collée, une portion curviligne en arc de cercle 22c collée sur la portion 2a et une portion rectiligne 22d non collée.

Comme on peut le voir sur la figure 5, les portions curvilignes collées respectives 21a, 22a et 21c, 22c définissent un tracé circulaire quasiment complet autour de la zone de la future ouverture de passage de jambes.

Après l'opération de retrait ou de rétraction par découpage des portions rectilignes non collées 21b, 22b et 22d, 21d, réalisée de la même manière que le découpe des portions rectilignes 18b et 19d dans le mode de réalisation de la figure 4, on pose des coussins absorbants 23 sur la feuille 2. Ces coussins, présentent une forme de sablier dont les bords circulaires latéraux viennent entourer la zone circulaire définie par les élastiques encollés 21 et 22 comme il vient d'être dit. On notera que sur la figure 5 on a représenté les portions rectilignes non collées 21d et 22d en traits interrompus à l'endroit du coussin 23 pour montrer leur emplacement avant retrait.

On découpe ensuite les ouvertures 12 qui présentent le même profil que dans les modes de réalisation précédents.

Bien que dans ce mode de réalisation on n'ait pas illustré de fil de déchirure, on comprendra qu'il serait parfaitement possible d'en prévoir comme précédemment.

La fabrication se termine, comme dans les modes de réalisation précédents par pliage autour de l'axe longitudinal X—X et découpe avec soudure simultanée le long des lignes transversales 10.

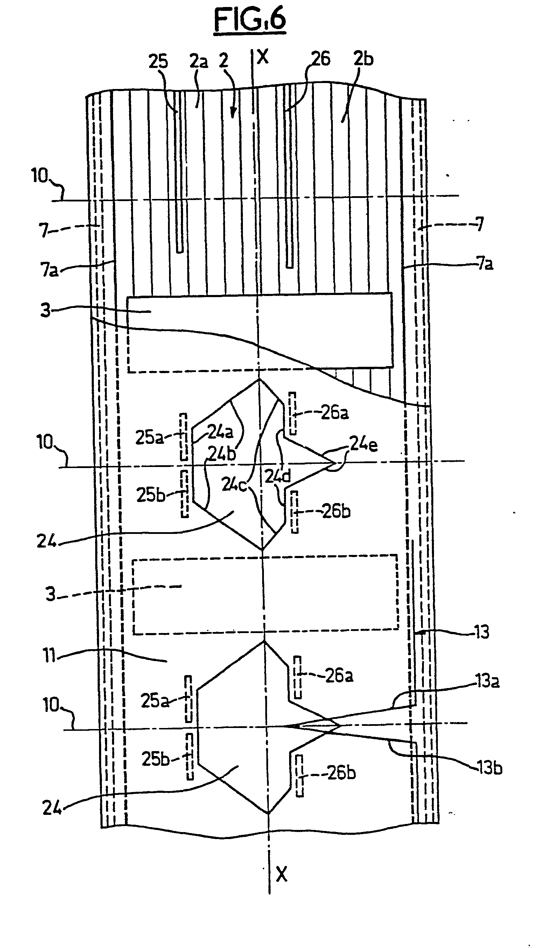

Dans le mode de réalisation de la figure 6 où les éléments identiques portent les mêmes références, les ouvertures de passage des jambes 24 présentent un profil constitué par une ligne brisée. Les élastiques d'entrejambe sont réalisés au moyen de deux élastiques continus 25 et 26 en forme de bandelettes encollées par intermittence à l'état tendu et selon un tracé rectiligne de part et d'autre de l'axe X—X. L'encollage des bandelettes élastiques 25 et 26 ne se fait que dans les portions 25a, 25b et 26a, 26b qui sont disposées symétriquement de chaque côté des lignes transversales respectives 10 et légèrement écartées les unes des autres en direction longitudinale.

Les portions non collées de bandelettes élastiques 25 et 26 restant entre ces zones collées, sont découpées et supprimées ou simplement relachées comme il a été dit pour les portions rectilignes 18b et 19d des bandelettes 18 et 19 du mode de réalisation de la figure 4, avant la pose des coussins absorbants 3.

Les ouvertures 24 ont une forme polygonale comportant un côté 24a parallèle à l'axe X—X définissant par ses portions situées de part et d'autre de la ligne transversale 10, les côtés avant des ouvertures de passage de jambe des couches-culottes finales et ce en combinaison avec les côtés 24b inclinés par rapport à la direction transversale 10.

Les bords arrière des ouvertures de passage de jambes sont définis par une ligne brisée constituée par les trois segments 24c, 24d et 24e, le segment 24d étant parallèle à l'axe longitudinal X—X et les deux segments respectifs 24e formant une pointe symétrique par rapport à la ligne 10.

Il en résulte que les portions élastiques 25a et 25b de la bandelette encollée 25 se trouvent disposées de chaque côté de la ligne 10 parallèlement et à proximité du côté 24a des ouvertures 24. De la même manière, les portions encollées 26a et 26b de la bandelette élastique 26 se trouvent disposées parallèlement et à proximité des segments rectilignes 24d des ouvertures 24 de chaque côté de la pointe formée par les segments 24e.

Un fil de déchirure 13 est encollé comme dans le mode de réalisation de la figure 2 après découpe des ouvertures 24 à l'intérieur desquelles pénètrent les différents tracés en V 13a, 13b.

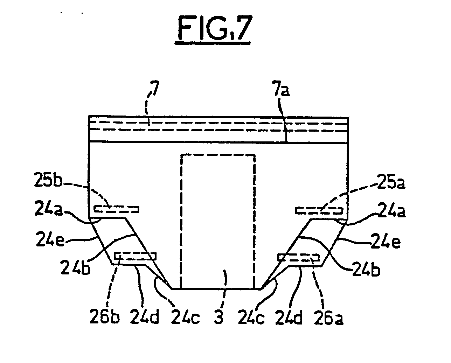

Après pliage autour de l'axe longitudinal X—X et découpe accompagnée d'une soudure autour de la ligne transversale 10, on obtient donc la couche-culotte représentée schématiquement en élévation sur la figure 7, et l'on note que les éléments élastiques 25a, et 25b, 26a, 26b réalisent un serrage élastique du passage des jambes améliorant l'étanchéité à cet endroit.

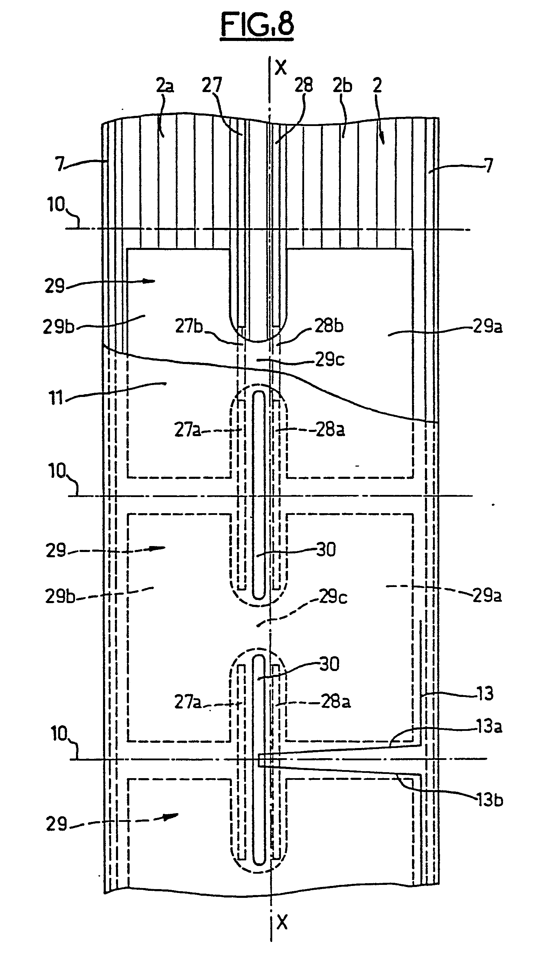

Dans le mode de réalisation de la figure 8 où les éléments identiques portent les mêmes références, les élastiques d'entre-jambes 27 et 28 sont collés par intermittence à l'état tendu et de façon rectiligne sur la feuille 2, de chaque côté de l'axe longitudinal X—X et à proximité de ce dernier.

On découpe comme dans le mode de réalisation de la figure 4, des portions non encollées 27b, 28b représentées en traits interrompus de façon à permettre la pose des coussins absorbant 29 qui affectent la forme d'un sablier, la portion arrière 29a sensiblement rectangulaire étant reliée à la portion avant 29b également rectangulaire par une mince zone de jonction 29c au droit de l'axe longitudinal X—X et à l'endroit où les portions 27b et 28b des élastiques 27 et 28 ont été retirées. On notera que la portion arrière 29a est de dimension légèrement supérieure à la portion avant 29b afin d'améliorer la forme anatomique de la couche-culotte terminée et grâce à une léger décalage de l'ensemble transversalement par rapport à l'axe longitudinal X—X.

Les ouvertures de passage de jambe affectent la forme de fentes longitudinales étroites 30 pratiquées de manière symétrique de chaque côté des lignes transversales 10, et entre les deux portions élastiques collées 27a et 28a. Après la réalisation de ces ouvertures 30 qui fait suite à la pose du voile perméable 11, un fil de déchirure 13 est collé sur la bande composite suivant le même tracé que dans le mode de réalisation de la figure 2, la pointe des V 13a, 13b pénétrant dans les ouvertures 30, les branches du V étant disposés de chaque côté de la ligne transversale 10.

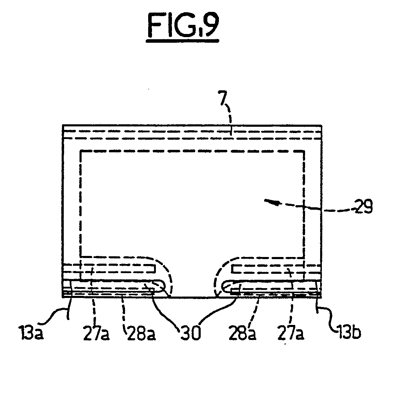

La couche-culotte est terminée comme précédemment par pliage autour de l'axe longitudinal X—X suivi d'une découpe accompagnée d'une soudure à chaud, le long de la ligne transversale 10. Dans cette opération de découpe, le fil de déchirure 13 est également sectionné, les extrémités des branches 13a et 13b apparaissant à l'extérieur de la couche-culotte comme on peut le voir sur la vue schématique en élévation de la figure 9 qui représente une telle couche-culotte à l'état terminé. Comme on peut le remarquer, les passages de jambes présentent des portions élastiques 27a, 28a sensiblement rectilignes parallèles à la bordure des passages de jambes elle-même parallèle à la ceinture équipée de l'élastique de ceinture 7.

Dans ce mode de réalisation où les ouvertures de jambes se trouvent dans un plan parallèle à la taille, il y a lieu de noter que la couche-culotte obtenue, telle que représentée sur la figure 9, est sensiblement de forme rectangulaire. Le périmètre des ouvertures de jambes est donc proche du périmètre de la taille. Compte tenu de l'anatomie des bébés, il est donc nécessaire que les passages des jambes soient munis des éléments élastiques précédemment décrits afin d'obtenir une réduction du périmètre du passage des jambes lorsque la couche-culotte est portée par le bébé.

En l'absence des éléments élastiques 27a et 28a, il serait nécessaire, dans ce mode réalisation, que la feuille imperméable 2, soit réalisée en un matériau extensible de façon à fournir par sa structure même un résultat identique.

Bien que l'invention ait été décrite à l'aide de quelques modes de réalisation à titre d'exemples, on notera que la plupart des particularités des différents modes de réalisation illustrés pourraient être appliquées aux autres modes de réalisation. Il en est ainsi notamment du mode de pose et d'utilisation du fil de déchirure dont les extrémités peuvent dans tous les modes de réalisation subsister dans la couche-culotte terminée au voisinage de la taille comme lors d'une fabrication selon le mode de réalisation de la figure 4, ou au contraire au voisinage du passage des jambes comme lors d'une fabrication selon les autres modes de réalisation illustrés.

De la même manière, la feuille perméable en non-tissé qui forme le revêtement interne de la couche-culotte, peut dans la plupart des modes de réalisation être posée avant ou après la formation d'un ourlet éventuel de ceinture recouvrant l'élastique de taille.

**Revendications**

1. Procédé de fabrication en continu de couches-culottes comprenant un coussin absorbant disposé entre une enveloppe extérieure imperméable à l'humidité et un voile intérieur perméable comprenant les étapes suivantes: on déroule une feuille souple continue (2) d'un matériau thermo-soudable imperméable à l'humidité; on colle à l'état étiré, sur au moins la majeure partie de la longueur de ladite feuille, un élastique continu (7); on pose sur la feuille imperméable (2), à intervalles réguliers, des coussins absorbants (3) de forme allongée, de manière que l'axe longitudinal des coussins soit perpendiculaire à l'axe longitudinal de ladite feuille; on déroule sur la feuille imperméable ainsi garnie de coussins, un voile continu (11) d'un matériau thermo-soudable mince, perméable à l'humidité de façon à obtenir une bande composite; on découpe dans ladite bande composite, entre chaque fois deux coussins successifs (3), entre les deux bords longitudinaux, une pluralité d'ouvertures (12) symétriques par rapport à une ligne transversale (10) passant perpendiculairement auxdits bords, à mi-distance entre les coussins successifs; on plie la bande composite précitée suivant une ligne longitudinale médian (X—X); et on sectionne, par une opération combinée de soudage et de découpage à chaud, la bande composite pliée, suivant la ligne transversale précitée.

2. Procédé suivant la revendication 1, caractérisé par le fait qu'avant le pliage, on applique sur le voile, de part et d'autre de la ligne transversale précitée, un fil de déchirure (13) en matière thermo-fusible, entre un bord longitudinal de la feuille et ladite ouverture.

3. Procédé suivant la revendication 2, caractérisé par le fait que l'on colle le fil de déchirure sur ladite bande composite.

4. Procédé suivant la revendication 2, caractérisé par le fait qu'on forme le fil de déchirure par extrusion d'un fil de matière thermoplastique en fusion sur ladite bande composite.

5. Procédé suivant l'une quelconque des revendications 1 à 4, caractérisé par le fait qu'on pose ledit fil de déchirure, avant de pratiquer lesdites ouvertures, au droit de chacune desdites ouvertures, suivant un tracé partant au-delà d'un bord longitudinal de la feuille, et s'étendant jusque dans ladite ouverture d'un côté de la ligne de sectionnement ultérieur, pour revenir au-delà dudit bord longitudinal de la feuille sur le côté opposé de ladite ligne transversale de sectionnement.

6. Procédé suivant l'une quelconque des revendications 1 à 4, caractérisé par le fait qu'on pose le fil de déchirure, après avoir pratiqué lesdites ouvertures, de façon continue sur le voile, suivant un tracé comprenant successivement, au droit de chaque ouverture, une partie partant en deçà d'un bord longitudinal de la feuille et s'étendant d'un côté de la ligne transversale de sectionnement ultérieur, jusque dans ladite ouverture pour revenir en-deçà dudit bord longitudinal de la feuille, du côté opposé de la ligne de sectionnement, suivie d'une partie rectiligne parallèle audit bord longitudinal, en-deçà de ce dernier.

7. Procédé suivant l'une quelconque des revendications 1 à 6, caractérisé par le fait qu'on colle lesdits élastiques continus (7), a faible distance des bords longitudinaux de la feuille imperméable, qu'on plie la partie marginale longitudinale de la feuille autour des élastiques et qu'on soude les bords pliés sur la feuille pour former deux ourlets (7a) contenant les deux élastiques continus.

8. Procédé suivant l'une quelconque des revendications 1 à 7, caractérisé par le fait qu'on dépose à chaud sur la feuille imperméable, autour du bord de chaque ouverture pratiquée ultérieurement une bande (9), d'un matériau thermoplastique en fusion capable de se rétracter lors de son refroidissement et possédant une bonne élasticité à l'état froid.

9. Procédé suivant l'une quelconque des revendications 1 à 7, caractérisé par le fait qu'avant de poser les coussins sur la feuille, on colle à l'état étiré deux èlastiques d'entre-jambe (18, 19), en forme de bandelettes sur la feuille imperméable de manière que lesdits élastiques entourent une partie au moins du bord de chacune des ouvertures pratiquées ultérieurement.

10. Procédé selon la revendication 9, caractérisé par le fait que l'on déplace périodiquement dans une direction transversale le point de contact initial de collage entre l'élastique d'entrejambe et la feuille en mouvement, de façon à obtenir un élastique d'entrejambes de forme incurvée, collé le long d'au moins une portion courbe de chaque ouverture de passage de jambe.

11. Procédé selon la revendication 10, caractérisé par le fait que le point de contact précité est maintenu fixe par intermittence, de façon à définir des portions rectilignes non collées destinées à être ultérieurement découpées et détendues ou supprimées.

12. Procédé suivant l'une quelconque des revendications 9 à 11, caractérisé par le fait qu'on utilise deux élastiques d'entrejambe (18, 19) continus et qu'on pose chaque élastique toujours d'un même côté desdites ouvertures par rapport à l'axe longitudinale de la bande composite.

13. Procédé suivant l'une quelconque des revendications 9 à 11, caractérisé par le fait qu'on utilise deux élastiques d'entrejambe continus (21, 22) et qu'on pose chaque élastique alternativement d'un côté d'une ouverture et du côté opposé de l'ouverture suivante, en croisant les deux élastiques entre les ouvertures.

14. Couche-culotte à jeter susceptible d'être fabriquée selon le procédé de l'une quelconque des revendications précédentes, comprenant une enveloppe extérieure (2) constituée par un matériau souple en feuille imperméable à l'humidité, un voile intérieur (11) perméable à l'humidité et un coussin absorbant (3) de forme allongée disposé entre ladite enveloppe extérieure et ledit voile intérieure, l'ensemble étant assemblé de façon que la couche-culotte présente la forme d'un slip fermé comportant un élastique (5) à la taille collé à l'intérieur de la feuille imperméable sur au moins la majeure partie du tour de taille, caractérisé par le fait que les côtés latéraux sont fermés par au moins soudure (4) s'étendant depuis la ceinture jusqu'à l'ouverture de passage des jambes, en disposant les deux côtés sensiblement bord à bord.

15. Couche-culotte suivant la revendication 14, caractérisée par le fait qu'elle comporte des éléments élastiques (6) disposés autour d'au moins une partie des ouvertures de passage pour les jambes.

16. Couche-culotte suivant l'une des revendications 14 ou 15, caractérisée par le fait qu'elle comporte au moins un fil de déchirure intérieur (4a) solidaire de la culotte et s'étendant depuis la taille jusqu'à l'une des ouvertures de passage pour les jambes.

17. Couche-culotte selon la revendication 15, caractérisée par le fait que le profil arrière (12b) de chaque ouverture de passage des jambes est courbe et convexe tandis que le profil avant (12a) est courbe et concave.

18. Couche-culotte selon la revendication 17, caractérisée par le fait que des éléments élastiques d'entrejambe (9a, 9b) suivent le profil des ouvertures de passage de jambes qu'ils entourent au moins sur une majeure partie de leur périphérie.

19. Couche-culotte selon la revendication 15, caractérisée par le fait que le profil arrière de chaque ouverture de passage de jambe est sensiblement convexe et délimité par une ligne brisée (24c, 24d, 24e) dont une section est parallèle à l'élastique de taille tandis que le profil avant est sensiblement concave et délimité par une ligne brisée (24a, 24b) dont une section est parallèle à l'élastique de taille.

20. Couche-culotte selon la revendication 19, caractérisée par le fait que les éléments élastiques d'entrejambe comprenent des portions d'élastiques en forme de bandelettes rectilignes (25, 26) collées le long des sections précitées parallèles à l'élastique de taille.

21. Couche-culotte selon l'une quelconque des revendications 14 à 20, caractérisée par le fait que le coussin absorbant est de forme sensiblement rectangulaire.

22. Couche-culotte selon l'une quelconque des revendications 14 à 20, caractérisée par le fait que le coussin absorbant affecte sensiblement la forme d'un sablier.

**Patentansprüche**

1. Verfahren zum kontinuierlichen, Herstellen von Windelhöschen, die ein saugfähiges Kissen aufweisen, das zwischen einer feuchtigkeitsundurchlässigen Außenhülle und einer durchlässigen Innenlage aufgenommen ist, welches die folgenden Stufen umfaßt: man wickelt eine kontinuierliche weiche Folie (2) eines warm verschweißbaren, feuchtigkeitsundurchlässigen Werkstoffes ab; man klebt im gespannten Zustand auf wenigstens den größeren Teil der Länge dieser Folie ein fortlaufendes elastisches Band (7); man legt auf die undurchlässige Folie (2) in regelmäßigen Abständen saugfähige Kissen (3) mit länglicher Form derart auf, daß die Längsachse der Kissen senkrecht zur Längsachse der Folie ausgerichtet ist; man rollt über die so mit Kissen ausgerüstete, undurchlässige Folie eine kontinuierliche Lage (11) eines dünnen, warm verschweißbaren Werkstoffes ab, der feuchtigkeitsdurchlässig ist, um ein zusammengesetztes Band zu erhalten; man schneidet in diesem zusammengesetzten Band zwischen jedem zweiten aufeinanderfolgenden Kissen (3) zwischen den beiden Längsrändern eine Vielzahl von Öffnungen (12) aus, die bezüglich einer Querlinie (10), die senkrecht zu den Rändern in der Mitte zwischen aufeinanderfolgenden Kissen verläuft, symmetrisch sind; man faltet das vorerwähnte zusammengesetzte Band längs einer Längsmittellinie (X—X); und man durchtrennt das gefaltete, zusammengesetzte Band in einem kombinierten Schweiß- und Heißschneideschritt längs der vorerwähnten Querlinie.

2. Verfahren nach Anspruch 1, dadurch gekennzeichnet, daß man vor dem Falten einerseits auf die Lage und anderseits auf die vorerwähnte Querlinie einen Reißfaden (13) aus warm schmelzbarem Werkstoff zwischen einem Längsrand der Folie und der erwähnten Öffnung anbringt.

3. Verfahren nach Anspruch 2, dadurch gekennzeichnet, daß man den Reißfaden auf das zusammengesetzte Band klebt.

4. Verfahren nach Anspruch 2, dadurch gekennzeichnet, daß man den Reißfaden durch Extrusion eines geschmolzenen, thermoplastischen Werkstoffes auf das zusammengesetzte Band bildet.

5. Verfahren nach einem der Ansprüche 1 bis 4, dadurch gekennzeichnet, daß man den Reißfaden vor dem Herstellen der Öffnungen rechts jeder der erwähnten Öffnungen längs einer Spur anbringt, die von jenseits eines Längsrandes der Folie ausgeht, sich bis über die erwähnte Öffnung von einer Seite der späteren Trennlinie erstreckt, um jenseits der erwähnten Längsrandes der Folie auf der gegenüberliegenden Seite der querverlaufenden Trennlinie zurückzukehren.

6. Verfahren nach einem der Ansprüche 1 bis 4, dadurch gekennzeichnet, daß man den Reißfaden nach dem Herstellen der Öffnungen derart kontinuierlich auf der Lage anbringt, daß er einer Spur folgt, die nacheinander rechts jeder Öffnung einen Teil aufweist, der diesseits eines Längsrandes der Folie ausgeht und sich von einer Seite der späteren, querverlaufenden Trennlinie bis in diese Öffnung erstreckt, um von der gegenüberliegenden Seite der Trennlinie diesseits des erwähnten Längsrandes der Folie zurückzukehren, gefolgt von einem geraden Teil, der parallel zum Längsrand diesseits von letzteren verläuft.

7. Verfahren nach einem der Ansprüche 1 bis 6, dadurch gekennzeichnet, daß man die kontinuierlichen elastischen Bänder in geringem Abstand von den Längsrändern der undurchlässigen Folie anklebt, daß man den Längsrandbereich der Folie um die elastischen Bänder herum faltet und daß man die herum gefalteten Ränder auf die Folie klebt, um zwei die kontinuierlichen, elastischen Bänder aufnehmende Säume zu bilden.

8. Verfahren nach einem der Ansprüche 1 bis 7, dadurch gekennzeichnet, daß man auf die undurchlässige Folie um den rand jeder später herzustellenden Öffnung im warmen Zustand ein Band (9) aus geschmolzenem, thermoplastischem Werkstoff ablegt, der in der Lage ist, sich während des Abkühlens zu verkürzen und der im kalten Zustand eine gute Elastizität besitzt.

9. Verfahren nach einem der Ansprüche 1 bis 7, dadurch gekennzeichnet, daß man vor dem Anbringen der Kissen auf der Folie im gespannten Zustand zwei elastische Zwischenbeinbänder (18, 19) in Form von Bändchen auf die undurchlässige Folie in der Weise anklebt, daß die elastischen Bänder wenigstens einen Teil des Randes jeder später hergestellten Öffnung umgeben.

10. Verfahren nach Anspruch 9, dadurch gekennzeichnet, daß man den ersten Klebeberührungspunkt zwischen dem elastischen Zwischenbeinband und der bewegten Folie periodisch in Querrichtung derart verlagert, um ein elastisches Zwischenbeinband mit einwärts gekrümmter Form zu erhalten, das längs wenigstens eines gekrümmten Teiles jeder Öffnung des Beindurchtrittes angeklebt ist.

11. Verfahren nach Anspruch 10, dadurch gekennzeichnet, daß der vorerwähnte Berührungspunkt intermittierend festgehalten ist, um so gerade, nicht angeklebte Bereiche zu bilden, die dazu bestimmt sind, später abgeschnitten und entspannt oder entfernt zu werden.

12. Verfahren nach einem der Ansprüche 9 bis 11, dadurch gekennzeichnet, daß man zwei elastische, kontinuierliche Zwischenbeinbänder (18, 19) verwendet und daß man jedes elastische Band stets auf der gleichen Seite der Öffnungen, bezogen auf die Längsachse des zusammengesetzten Bandes, anbringt.

13. Verfahren nach einem der Ansprüche 9 bis 11, dadurch gekennzeichnet, daß man zwei elastische, kontinuierliche Zwischenbeinbänder (21, 22) verwendet und daß man jedes elastische Band alternierend auf der einen Seite einer Öffnung und der gegenüberliegenden Seite der nachfolgenden Öffnung anbringt, wobei sich die elastischen Bänder zwischen den Öffnungen kreuzen.

14. Wegwerfwindelhöschen, das nach dem Verfahren gemäß einem der vorhergehenden Ansprüche hergestellt werden kann, umfassend eine Außenhülle (2), die durch einen weichen, feuchtigkeitsundurchlässigen Folienwerkstoff gebildet ist, eine feuchtigkeitsdurchlässige Innenlage (11) und ein saugfähiges Kissen (3) mit länglicher Form, das zwischen der Außenhülle und der Innenlage angeordnet ist, wobei die Anordnung so zusammengesetzt ist, daß das Windelhöschen die Form eines geschlossenen Slip aufweist, der ein elastisches Band (5) im Taillenbereich aufweist, das auf der Innenseite der undurchlässigen Folie wenigstens über den größeren Teil des Umfanges der Taillie angeklebt ist, dadurch gekennzeichnet, daß die beiden Längsseiten durch wenigstens eine Schweißung (4) verbunden sind, die sich vom Gürtel bis zur Beindurchtrittsöffnung erstreckt, wobei die beiden Seiten im wesentlichen stumpf angeordnet sind.

15. Windelhöschen nach Anspruch 14, dadurch gekennzeichnet, daß es elastische Elemente (6) aufweist, die um wenigstens einen Teil der Beindurchtrittsöffnung herum angeordnet sind.

16. Windelhöschen nach den Ansprüchen 14 oder 15, dadurch gekennzeichnet, daß es wenigstens einen inneren Reißfaden (4a) aufweist, der mit dem Höschen verbunden ist und sich von der Taillie bis zu einer der Beindurchtrittsöffnungen erstreckt.

17. Windelhöschen nach Anspruch 15, dadurch gekennzeichnet, daß das hintere Profil (12b) jeder Beindurchtrittsöffnung gekrümmt und konvex ist, wogegen das vordere Profil (12a) gekrümmt und konkav ist.

18. Windelhöschen nach Anspruch 17, dadurch gekennzeichnet, daß die elastischen Zwischenbeinelemente (9a, 9b) dem Profil der Durchtrittsöffnungen für die Beine folgen, die sie wenigstens über den größeren Teil ihres Umfanges umgeben.

19. Windelhöschen nach Anspruch 15, dadurch gekennzeichnet, daß das hintere Profil jeder Beindurchtrittsöffnung im wesentlichen konvex und durch eine punktierte Linie (24c, 24d, 24e) begrenzt ist, von welcher ein Abschnitt parallel zum elastischen Taillenband verläuft, wogegen das vordere Profil im wesentlichen konkav und durch eine punktierte Linie (24a, 24b) begrenzt ist, von welcher ein Abschnitt parallel zum elastischen Taillenband verläuft.

20. Windelhöschen nach Anspruch 19, dadurch gekennzeichnet, daß die elastischen Zwischenbeinelemente elastische Abschnitte in Form von geraden Bändchen (25, 26) umfassen, die längs der vorerwähnten, zum elastischen Taillenband parallelen Abschnitte angeklebt sind.

21. Windelhöschen nach einem der Ansprüche 14 bis 20, dadurch gekennzeichnet, daß das saugfähige Kissen im wesentlichen rechteckförmig ist.

22. Windelhöschen nach einem der Ansprüche 14 bis 20, dadurch gekennzeichnet, daß das saugfähige Kissen im wesentlichen die Form einer Sanduhr annimmt.

**Claims**

1. Process for a continuous manufacture of diapers comprising an absorbent pad arranged between a moisture-proof external cover and a permeable internal liner comprising the following steps: a continuous flexible sheet (2) of a moistureproof, heat-weldable material is unrolled; a continuous elastic (7) in a stretched state is glued to at least the greater part of the length of the said sheet; absorbent pads (3) of elongated shape are placed on the moistureproof sheet (2) at uniform intervals, so that the lengthwise axis of the pads is perpendicular to the lengthwise axis of the said sheet; a continuous liner (11) of a thin, moisture-permeable and heat-weldable material is unrolled onto the moisture-proof sheet provided with pads in this manner, so as to form a composite strip; a plurality of openings (12) which are symmetrical relative to a transverse line (10) passing at right angles to both lengthwise edges, midway between the successive pads, are cut out from the said composite strip, between each two successive pads (3) between the two said edges; the abovementioned composite strip is folded along a center lengthwise line (X—X); and the folded composite strip is severed along the abovementioned transverse line by a combined hot welding and cutting operation.

2. Process according to Claim 1, characterised in that before the folding, a tearing thread (13) made of thermofusible material is applied onto the liner, on both sides of the abovementioned transverse line, between lengthwise edge of the sheet and the said opening. ·

3. Process according to Claim 2, characterised in that the tearing thread is glued to the said composite strip.

4. Process according to Claim 2, characterised in that the tearing thread is formed by extruding a thread of molten thermoplastic material onto the said composite strip.

5. Process according to any one of Claims 1 to 4, characterised in that, before the said openings are produced, the said tearing thread is placed at right angles to each of the said openings, along a line starting beyond a lengthwise edge of the sheet and extending into the said opening on one side of the subsequent severing line, to return beyond the said lengthwise edge of the sheet on the opposite side of the said transverse severing line.

6. Process according to any one of Claims 1 to 4, characterised in that, after the said openings have been produced, the tearing thread is placed continuously on the liner, along a line comprising successively, at right angles to each opening, a section starting on the inner side of a lengthwise edge of the sheet and extending from one side of the transverse line of subsequent severing, into the said opening, to return to the innerside of the said lengthwise edge of the sheet, on the opposite side of the severing line, followed by a rectilinear section parallel to the said lengthwise edge, on the inner side of the latter.

7. Process according to any one of Claims 1 to 6, characterised in that the said continuous elastics (7) are glued at a small distance from the lengthwise edges of the moistureproof sheet, that the lengthwise marginal section of the sheet is folded around the elastics and that the folded edges are welded onto the sheet, forming two hems (7a) enclosing the two continuous elastics.

8. Process according to any one of Claims 1 to 7, characterised in that a strip (9) of molten thermoplastic material capable of shrinking when cooling and having good elasticity when cold is deposited hot on the moistureproof sheet, around the edge of each opening subsequently produced.

9. Process according to any one of Claims 1 to 7, characterised in that before the pads are placed on the sheet, two crotch elastics (18, 19) are glued in a stretched state, in the shape of narrow strips, onto the moistureproof sheet so that the said elastics surround at least part of the edge of each of the openings subsequently produced.

10. Process according to Claim 9, characterised in that the initial point of contact of gluing between the crotch elastic and the moving sheet is moved at regular intervals in a transverse direction, so as to produce a curved crotch elastic glued along at least a curved portion of each leg-passage opening.

11. Process according to Claim 10, characterised in that the abovementioned point of contact is intermittently kept stationary, so as to define unglued rectilinear sections intended to be subsequently cut out and slackened or removed.

12. Process according to any one of Claims 9 to 11, characterised in that two continuous crotch elastics (18, 19) are used and that each elastic is placed constantly on the same side of the said openings relative to the lengthwise axis of the composite strip.

13. Process according to any one of Claims 9 to 11, characterised in that two continuous crotch elastics (21, 22) are used and that each elastic is placed alternately on one side of an opening and on the opposite side of the following opening, by crossing the two elastics between the openings.

14. Disposable diaper capable of being manufactured according to the process of any one of the preceding claims, comprising an outer cover (2) consisting of a moisture-proof flexible sheet material, a moisture-permeable inner liner (11) and an absorbent pad (3) of elongated shape arrange between the said outer cover and the said inner liner, the whole being assembled so that the diaper is in the shape of closed pants incorporating an elastic (5) at the waist, glued to the inside of the moistureproof sheet over at least the greater part of the waistline, characterised in that the side edges are closed by at least one weld (4) extending from the belt down to the leg passage opening, the two sides being placed substantially edge to edge.

15. Diaper according to Claim 14, characterised in that they incorporate elastic members (6) arranged around at least a part of the leg passage openings.

16. Diaper according to either of Claims 14 or 15, characterised in that it incorporates at least one internal tearing thread (4a) attached integrally to the pants and extending from the waist to one of the leg passage openings.

17. Diaper according to Claim 15, characterised in that the rear contour (12b) of each leg passage opening is curved and convex, while the front contour (12a) is curved and concave.

18. Diaper according to Claim 17, characterised in that elastic crotch members (9a, 9b) follow the contour of the leg passage openings which they surround over at least a greater part of their circumference.

19. Diaper according to Claim 15, characterised in that the rear contour of each leg passage opening is substantially convex and defined by a broken line (24c, 24d, 24e) a part of which is parallel to the waist elastic while the front contour is substantially concave and defined by a broken line (24a, 24b) a part of which is parallel to the waist elastic.

20. Diaper according to Claim 19, characterised in that the crotch elastic members comprise sections of elastics in the shape of narrow rectilinear strips (25, 26) glued along the abovementioned parts which are parallel to the waist elastic.

21. Diaper according to any one of Claims 14 to 20, characterised in that the absorbent pad is of a substantially rectangular shape.

22. Diaper according to any one of Claims 14 to 20, characterised in that the absorbent pad is substantially hourglass-shaped.

# FIG.1

# FIG.3

# FIG.2

0 048 011

FIG.4

3

# FIG.5

# FIG.6

# FIG.7

# FIG.8

# FIG.9